(19) Europäisches Patentamt
European Patent Office
Office européen des brevets

(11) Veröffentlichungsnummer: **0 062 853**
**B1**

(12) # EUROPÄISCHE PATENTSCHRIFT

(45) Veröffentlichungstag der Patentschrift: **06.11.85**

(51) Int. Cl.⁴: **C 07 D 249/12**

(21) Anmeldenummer: **82102794.3**

(22) Anmeldetag: **02.04.82**

(54) **Verfahren zur Herstellung von substituierten 3-Hydroxy-1,2,4-triazolen.**

(30) Priorität: **09.04.81 DE 3114314**
**09.04.81 DE 3114316**

(43) Veröffentlichungstag der Anmeldung:
**20.10.82 Patentblatt 82/42**

(45) Bekanntmachung des Hinweises auf die Patenterteilung:
**06.11.85 Patentblatt 85/45**

(84) Benannte Vertragsstaaten:
**AT BE CH DE FR GB IT LI NL SE**

(56) Entgegenhaltungen:
**DE - A - 2 537 973**
**DE - A - 2 634 854**
**US - A - 3 625 951**

(73) Patentinhaber: **HOECHST AKTIENGESELLSCHAFT,**
**Postfach 80 03 20, D-6230 Frankfurt am Main 80 (DE)**

(72) Erfinder: **Koch, Manfred, Dr., Kreuzheck 2,**
**D-6230 Eppstein/Taunus (DE)**
Erfinder: **Stähler, Gerhard, Dr., Loreleistrasse 87,**
**D-6230 Frankfurt am Main 80 (DE)**

**EP 0 062 853 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

3-Hydroxytriazole der Formel I sind bekannte Zwischenprodukte zur Herstellung von Pflanzenschutzmitteln (vgl. DBP 910 652, 1 299 924, 1 670 876, DE-OS 2 251 074, 2 251 075, 2 251 096, 2 352 141, 2 352 142).

Es ist bekannt, 1-Phenyl-3-hydroxy-1,2,4-triazol durch Kochen von 1-Phenylsemicarbazid mit Ameisensäure herzustellen (O. Widmann, Chem. Ber. 26 (1893), 2613), jedoch liegt die Ausbeute trotz Anwendung eines grossen Überschusses von Ameisensäure und langer Reaktionsdauer noch unter 50% d.Th. und ist somit wenig befriedigend.

Es ist ferner bekannt, die Verbindungen der Formel I durch Umsetzung von entsprechenden Semicarbaziden mit Orthoameisensäureester zu erhalten (DE-OS 2 251 074).

Es wurde nun gefunden, dass man die Ausbeute – bei wesentlich verkürzter Reaktionszeit – erheblich steigern kann, wenn man die Umsetzung mit Ameisensäure unter Zusatz von anorganischen oder starken organischen Säuren bzw. deren Halogeniden oder Anhydriden durchführt.

Gegenstand der vorliegenden Erfindung ist daher ein Verfahren zur Herstellung von in 1-Stellung substituierten 3-Hydroxy-1,2,4-triazolen durch Umsetzung von gegebenenfalls substituierten Phenylhydrazin(salzen) mit Harnstoff oder Alkali- bzw. Ammoniumcyanaten und Cyclisierung der erhaltenen Phenylsemicarbazide mittels Ameisensäure, dadurch gekennzeichnet, dass man die Cyclisierung in Gegenwart einer anorganischen Säure oder eines Halogenids derselben vornimmt.

Nach dem erfindungsgemässen Verfahren erhält man in 1-Stellung substituierte 3-Hydroxytriazole der Formel

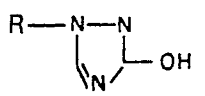

I

worin R gegebenenfalls substituiertes Phenyl bedeutet. Die Art des Substituenten von R ist für das Gelingen der Reaktion ohne Belang, wenn man davon absieht, dass R sich unter den Reaktionsbedingungen inert verhalten muss und die entsprechenden Semicarbazide der Formel

$R\text{–}NHNH\text{–}CONH_2$ II

zugänglich sein müssen. Bevorzugt bedeutet R einen ggf. durch ein oder zwei Substituenten aus der Gruppe $(C_1\text{–}C_4)$-Alkyl, $(C_1\text{–}C_4)$-Alkoxy, $(C_1\text{–}C_4)$-Alkylthio, Halogen, Trifluormethyl, Cyano, Nitro und $(C_1\text{–}C_4)$-Alkoxycarbonyl substituiertes Phenyl bedeutet, cyclisiert.

Zur Erzielung hoher Ausbeuten benötigt man mindestens die stöchiometrisch benötigte Menge von 1 mol, vorzugsweise 2 bis 3 mol Ameisensäure pro mol Semicarbazid (II). Grössere Mengen sind unschädlich, bringen aber auch keine Vorteile. Die Ameisensäure kann unverdünnt eingesetzt werden; vorzugsweise wird zur Umsetzung jedoch ein inertes Lösungs- oder Verdünnungsmittel zugesetzt. Als solche können beispielsweise verwendet werden: Wasser, Kohlenwasserstoffe wie Toluol oder Xylol, Chlorkohlenwasserstoffe wie Chlorbenzol oder Tetrachlorkohlenstoff, Carbonsäurenitrile wie Propionitril oder Carbonsäureamid wie Dimethylformamid. Besonders bevorzugt ist Wasser. Die Menge des inerten Lösungs- oder Verdünnungsmittels liegt zwischen null und 500 ml pro mol Semicarbazid. Die Ameisensäure im Reaktionsgemisch kann durch den Zusatz des Lösungs- oder Verdünnungsmittels bis zum zehnfachen des ursprünglichen Volumens verdünnt werden; noch grössere Verdünnungen sind nicht zu empfehlen. Verwendet man Wasser als Lösungsmittel oder Verdünnungsmittel, so wird ein Wassergehalt von 15–85%, insbesondere 50–80% bevorzugt.

Als Säure können an sich alle bekannten anorganischen Säuren verwendet werden, insbesondere Schwefelsäure, Salzsäure, Chlorwasserstoffgas und Phosphorsäure. Als Säurehalogenide kommen vorzugsweise anorganische Säurechloride und -bromide in Betracht, die sich mit vorhandenem Wasser zu Säuren zersetzen. Beispielsweise verwendbar: Sulfurylchlorid, Phosphortrichlorid oder -tribromid, Phosphorpentachlorid und Phosphoroxychlorid. Die Menge dieser sauren Zusätze beträgt vorzugsweise 0,05 bis 0,5 mol, insbesondere 0,1–0,3 mol pro mol Semicarbazid.

Die Reaktionstemperatur soll oberhalb 80 °C liegen und kann beim Arbeiten in druckfesten Gefässen bis zu 150 °C betragen. Vorzugsweise wird die Umsetzung bei 90–100 °C drucklos durchgeführt. Die Reaktionszeit liegt je nach der angewandten Menge von Ameisensäure und anorganischer Säure zwischen einer Stunde und vierundzwanzig Stunden. Die Isolierung der Reaktionsprodukte kann in einfacher Weise durch Absaugen des ausgefallenen Triazols erfolgen; wird bei höherem Gehalt der wässrigen Ameisensäure gearbeitet, empfiehlt es sich, den Reaktionsansatz mit weiterem Wasser zu verdünnen.

Aus ökologischer und betriebswirtschaftlicher Sicht ist eine hohe Ausnutzung der bevorzugt im Überschuss eingesetzten Ameisensäure notwendig. Das erfindungsgemässe Verfahren gestattet es, die gesamte nach dem Abfiltrieren des Reaktionsprodukts anfallende Mutterlauge für weitere Ansätze zu verwenden, wobei jeweils nur der Verbrauch an Semicarbazid, Ameisensäure und anorganischer Säure ersetzt zu werden braucht. Gewünschtenfalls kann man die Ameisensäure auch aus der Mutterlauge durch Zugabe eines Alkohols als Ester abdestillieren (z.B. als Methylformiat). Eine andere Möglichkeit der Rückgewinnung ist die azeotrope Destillation, z.B. mit Hilfe des Azeotrops Ameisensäure/Wasser oder Ameisensäure/Xylol/Wasser. Auch eine extraktive Entfernung aus wässrigen Ameisensäurelösungen mit einem hauptsächlich Ameisensäure aufnehmenden Extraktionsmittel, wie z.B. N,N-Dibutylform-

amid, ist möglich. Aus diesem kann Ameisensäure durch Destillation zurückerhalten werden.

Das erfindungsgemässe Verfahren bietet ferner den Vorteil, dass es mit der Herstellung der Phenylsemicarbazide II zu einem Eintopfverfahren kombiniert werden kann. Phenylsermicarbazide werden durch Umsetzung der entsprechenden Phenylhydrazinhydrochloride oder -acetate mit Kaliumcyanat in Wasser (Ann. 190, 113; Ber. 25, 2613) oder durch Umsetzung der freien Phenylhydrazine oder ihrer Hydrochlorids mit Harnstoff (Gazz. chim. Ital. 16, 202; Ber. 20, 2359) hergestellt. Bisher war es erforderlich, das in erster Stufe erhaltene Phenylsemicarbazid II für die weitere Umsetzung zu I zu isolieren und zu reinigen. Dies ist von Nachteil, da das Phenylhydrazin nicht quantitativ reagiert, so dass bei der Aufarbeitung des Phenylsemicarbazids nicht verbrauchtes Phenylhydrazin in das Abwasser gelangt. Letzteres ist als Blutgift für Warmblüter bekannt, hoch fischtoxisch und wegen seiner bakteriziden Eigenschaften nur schwer biologisch abzubauen. Die Entfernung des Phenylhydrazins aus dem Abwasser bis auf unschädliche Konzentrationen ist daher nur auf sehr umständlichem Wege möglich.

Nach einer Variante des erfindungsgemässen Verfahrens kann nun die Herstellung des Phenylsemicarbazids nach den bekannten Verfahren mit dessen weiterer Umsetzung zum Phenyltriazol kombiniert werden, indem man zunächst ein Phenylhydrazin entweder mit einer mindestens äquimolaren Menge, vorzugsweise mit einem geringen Überschuss von bis zu 15 Mol-% eines cyansauren Salzes (NaOCN, KOCN, $NH_4OCN$) in Gegenwart einer etwa äquimolaren Menge einer organischen oder anorganischen Säure bei 0°–60 °C oder mit einer mindestens äquimolaren Menge, vorzugsweise einem Überschuss von bis zu 40 Mol-%, Harnstoff in Gegenwart von ca. 0,001–1,35 Mol, bevorzugt 0,1–1,35 Mol (bezogen auf eingesetztes Phenylhydrazin der Formel II) einer organischen oder anorganischen Säure oder deren Ammoniumsalz bei 100°–160 °C, vorzugsweise 105°–140 °C, in einem der vorgenannten Lösungsmittel, gegebenenfalls unter Druck, zu II umsetzt und das Reaktionsgemisch nach Zugabe von Ameisensäure und weiterer anorganischer Säure in der beschriebenen Weise zu I reagieren lässt.

Durch die Zusammenfassung der beiden Verfahrensstufen wird die Gesamtausbeute verbessert und Abwasserbelastungen vermindert.

Das erfindungsgemässe Verfahren liefert die Hydroxytriazole der Formel I in ausgezeichneter Ausbeute und hoher Reinheit, die für die meisten Verwendungszwecke – z.B. für die Umsetzung zu Pflanzenschutzmitteln – eine nachträgliche Reinigung überflüssig macht. Das Arbeiten in wässriger oder wässrig-organischer Lösung hat den zusätzlichen Vorteil, dass man technische (ca. 85%ige) Ameisensäure verwenden kann und die – gewöhnlich aus wässriger Suspension isolierten – Semicarbazide vor dem Ringschluss nicht zu trocknen braucht.

Beispiel 1

1-Phenyl-3-hydroxy-1,2,4-triazol

151 g 1-Phenylsemicarbazid, 135,3 g (2,5 mol) 85%ige Ameisensäure (techn.) und 25 g (0,25 mol) konzentrierte Schwefelsäure werden sechs Stunden bei 95–100 °C gerührt. Die entstandene, viskose Masse wird mit 400 ml Wasser verdünnt, abgekühlt und der Niederschlag von 1-Phenyl-1,2,4-triazolon-3 abfiltriert und mit Wasser neutral gewaschen. Nach Trocknen bei 100°, 200 mb werden 145,5 g = 90,3% d.Th. 1-Phenyl-1,2,4-triazolon-3 vom Schmelzpunkt 285–286 °C erhalten.

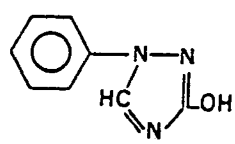

Beispiel 2–17

In gleicher Versuchsdurchführung, jedoch mit geänderter Ansatzgrösse, Reaktionszeit und Ameisensäurekonzentration wurden nachfolgend in Tabelle 1 aufgeführte Versuche ausgeführt und 1- Phenyl -3- hydroxy - (1,2,4) - triazol erhalten. Eingesetzt wurde technisches trockenes Phenylsemicarbazid.

Tabelle 1 Erläuterungen:
PSC = Phenylsemicarbazid,
Ausbeute: bezogen auf PSC techn., $H_2O$: einschliesslich der in Ameisensäure techn. enthaltenen Wassermenge.

Tabelle I Beispiele 2–17 1-Phenyl-2-hydroxy-1,2,4-triazol

| Beispiel Nr. | Ansatz PSC [mol] | HCOOH [mol] | $H_2O$ [ml] | $H_2SO_4$ [mol] | Ameisen säuregehalt der wässrigen Lösung | Reaktions- zeit [h] | temp. [°C] | Ausbeute [% d.Th.] | Fp. [°C] |
|---|---|---|---|---|---|---|---|---|---|
| 2 | 1.0 | 2.0 | – | 0.25 | 100% | 3 | 110 | 86.5 | 286-7 |
| 3 | 1.0 | 2.5 | 20 | 0.25 | 85% | 4 | 110 | 89.5 | 285-6 |
| 4 | 1.0 | 2.5 | 20 | 0.10 | 85% | 24 | 110 | 90.6 | 289-91 |
| 5 | 1.0 | 2.5 | 20 | 0.15 | 85% | 6 | 110 | 90.3 | 287.9 |
| 6 | 1.0 | 3.0 | – | 0.25 | 100% | 6 | 110 | 87.4 | 284-5 |
| 7 | 1.0 | 2.5 | 45 | 0.25 | 71.9% | 6 | 105 | 90.3 | 285-7 |

Tabelle I (Fortsetzung) Beispiele 2–17 1-Phenyl-2-hydroxy-1,2,4-triazol

| Beispiel Nr. | Ansatz PSC [mol] | HCOOH [mol] | $H_2O$ [ml] | $H_2SO_4$ [mol] | Ameisensäuregehalt der wässrigen Lösung | Reaktions- zeit [h] | Reaktions- temp. [°C] | Ausbeute [% d.Th.] | Fp. [°C] |
|---|---|---|---|---|---|---|---|---|---|
| 8 | 1.0 | 2.5 | 85 | 0.25 | 57.5% | 16 | 105 | 92.0 | 285-6 |
| 9 | 1.0 | 2.5 | 85 | 0.25 | 57.5% | 4 | 105 | 91.0 | 283-4 |
| 10 | 1.0 | 2.5 | 320 | 0.25 | 26.4% | 16 | 100 | 91.8 | 285-6 |
| 11 | 1.0 | 2.5 | 320 | 0.25 | 26.4% | 4 | 100 | 90.0 | 285-6 |
| 12 | 1.0 | 2.5 | 520 | 0.25 | 18.1% | 16 | 100 | 89.7 | 285-6 |
| 13 | 1.0 | 2.5 | 270 | 0.25 | 40.3% | 4 | 95–100 | 89.8 | 283 |
| 14 (Vgl.) | 1.0 | 2.5 | 20 | – | 85% | 6 | 95–100 | 8.7 | 282-4 |
| 15 | 1.0 | 2.0 | 270 | 0.25 | 40.3% | 4 | 95–100 | 87.6 | 285-6 |
| 16 | 1.0 | 1.3 | 270 | 0.25 | 40.3% | 24 | 95–100 | 86.5 | 280-2 |
| 17 | 1.0 | 1.5 | 270 | 0.25 | 40.3% | 16 | 95–100 | 88.6 | 280-2 |

Beispiel 18

450,75 g (2,018 mol) wasserfeuchtes techn. 1-Phenylsemicarbazid (Feststoffgehalt 67,6%), 300 ml Wasser, 270,6 g (5 mol) 85%ige Ameisensäure techn. und 50 g (0,5 mol) konzentrierte Schwefelsäure werden vier Stunden bei 95 °C gerührt. Die Reaktionslösung wird im Vakuum abfiltriert und die anfallende Mutterlauge, die neben unumgesetztem 1-Phenylsemicarbazid hauptsächlich Ameisensäure und Schwefelsäure enthält, für einen weiteren Ansatz separiert. Anschliessend wird der Filterkuchen, bestehend aus 1-Phenyl-3-hydroxytriazol mit Wasser neutral gewaschen und im Vakuumtrockenschrank getrocknet. Man erhält 292,3 g (89,95% d.Th.) 1-Phenyl-3-hydroxytriazol mit dem Schmelzpunkt 284–285 °C.

Beispiel 19

151 g 1-Phenylsemicarbazid, 108,25 g (2,0 mol) 85%ige Ameisensäure techn., 65 ml Wasser und 25 g (0,25 mol) Schwefelsäure werden acht Stunden bei 100 °C gerührt. Anschliessend lässt man abkühlen und tropft 41,6 g (1,3 mol) Methanol zur Reaktionsmischung zu, wobei Methylformiat (Kp. 32 °C) über eine Kolonne abdestilliert wird. Man belässt die Reaktionsmischung bei etwa 50–60 °C, bis die Methylformiatbildung beendet ist. Anschliessend wird mit 400 ml Wasser versetzt, abfiltriert und der Filterkuchen mit Wasser neutral gewaschen und im Vakuum getrocknet. Man erhält 146,2 g (90,8% d.Th.) 1-Phenyl-3-hydroxytriazol vom Schmelzpunkt 286–287 °C.

Beispiel 20

185,5 g (1,0 mol) 1-(4-Chlorphenyl)- semicarbazid, 135,2 g (2,5 mol) 85%ige Ameisensäure, 40 ml Wasser und 30 g (0,3 mol) konzentrierte Schwefelsäure werden 6 Stunden bei 95 °C erhitzt. Anschliessend werden 400 ml Wasser hinzugefügt, die Reaktionsmischung wird abfiltriert, der Filterkuchen mit Wasser neutral gewaschen und im Vakuumtrockenschrank (100 °C, 200 mb) getrocknet. Man erhält 170 g (87% d.Th.) 1-(4-Chlorphenyl)-3- hydroxytriazol vom Schmelzpunkt 305 °C.

Tabelle 2 Beispiele 21–32

Analog Beispiel 20 wurden folgende Verbindungen der allgemeinen Formel I hergestellt:

| Beispiel Nr. | R | Ausbeute [% d.Th.] | Fp. [°C] |
|---|---|---|---|
| 21 | 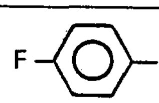 | 88,6 | 298–300 |
| 22 | 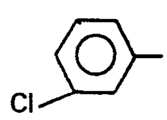 | 91,2 | 306 |

Tabelle 2 (Fortsetzung)

| Bei-spiel Nr. | R | Ausbeute [% d.Th.] | Fp. [°C] |
|---|---|---|---|
| 23 | 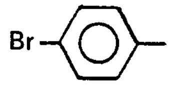 | 89,4 | 222–25 |
| 24 | 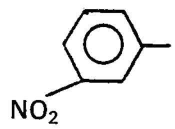 | 86,2 | 293–295 |
| 25 | 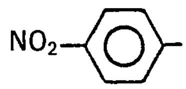 | 87,8 | 324 |
| 26 | 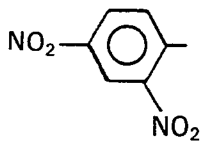 | 85,9 | 162–164 |
| 27 | 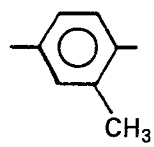 | 89,1 | 169–170 |
| 28 | 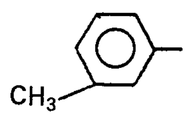 | 88,7 | 238–240 |
| 29 | 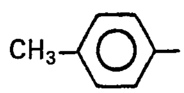 | 89,9 | 297 |
| 30 | 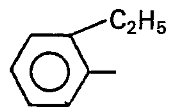 | 90,2 | 190–192 |
| 31 | 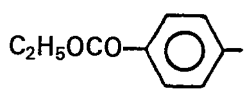 | 86,1 | 160 |
| 32 | 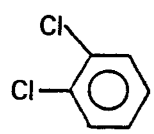 | 90,3 | 303–305 |

Beispiel 33

Zu einer Lösung von 151 g (1 mol) 1-Phenylsemicarbazid in 135,2 g (2,5 mol) 85%iger Ameisensäure und 65 ml Wasser gibt man vorsichtig 36,5 g konzentrierte (30%ige) Salzsäure (0,3 mol) hinzu und erhitzt 4 Stunden bei 95 °C. Anschliessend wird mit 200 ml Wasser verdünnt und abfiltriert. Der Filterkuchen wird mit Wasser neutral gewaschen und getrocknet. Man erhält 142 g (88,2 g % d.Th.) 1-Phenyl-3-hydroxytriazol vom Schmelzpunkt 282–283 °C.

Beispiel 34 (Eintopfverfahren)

1-Phenyl-3-hydroxy-1,2,4-triazol

225 g (2 mol) Phenylhydrazin technisch 96%ig, 221,2 g (2 mol) 33%ige Salzsäure, 120 g (2 mol) Harnstoff und 800 ml Wasser werden im geschlossenen Gefäss 5 Stunden auf 125–128 °C erhitzt. Hierbei entsteht ein Druck von 2,6–2,8 at.

Anschliessend wird auf 100 °C abgekühlt und 324,7 g (6 mol) 85%ige Ameisensäure und 50 g (0,5 mol) konzentrierte Schwefelsäure tropfenweise zugegeben; anschliessend wird 6 Stunden

bei 95 °C gerührt, abgekühlt und das ausgefallene 1-Phenyl-3-hydroxy-1,2,4-triazol abgesaugt. (Aus der Mutterlauge kann nach Zugabe von 144 g Methanol (4,5 mol) der Ameisensäureüberschuss als Methylformiat durch Abdestillieren (Sp. 32°) zurückgewonnen werden).

Der Filterkuchen wird reichlich mit Wasser gewaschen und im Vakuum bei 100 °C getrocknet. Man erhält 238,3 g (74% d.Th.) 1-Phenyl- 3-hydroxytriazol vom Fp. 280–282 °C.

Beispiel 35 (Eintopfverfahren)
1-Phenyl-3-hydroxy-1,2,4-triazol

108 g (1 mol) Phenylhydrazin und 60 g (1 mol) Harnstoff werden in 500 ml Xylol suspendiert und unter gutem Rühren 111 g (1 mol) konzentrierte Salzsäure zugegeben. Anschliessend wird 2,5 Stunden auf 135 °C erhitzt und dabei gebildetes Wasser über einen Wasserabschneider abgetrennt.

Nach Abkühlen auf 90 °C gibt man zunächst 135,2 g (2,5 mol) 85%ige Ameisensäure, dann 25 g (0,25 mol) konzentrierte Schwefelsäure zu und erhitzt weiter 6 Stunden bei 95 °C. Nach Abkühlen wird das Endprodukt abfiltriert, gründlich mit Wasser säurefrei gewaschen und im Vakuum bei 100 °C getrocknet. Man erhält 124,6 g (77,5% d.Th.) 1-Phenyl-3-hydroxy-1,2,4-triazol mit dem Fp. 281–282 °C.

Beispiel 36 (Eintopfverfahren)
1-Phenyl-3-hydroxy-1,2,4-triazol

108 g (1 mol) Phenylhydrazin, 60 g (1 mol) Harnstoff und 300 ml Xylol werden in einen emaillierten Autoklaven gegeben. Anschliessend wird unter gutem Rühren 5,5 g (0,15 mol) Chlorwasserstoff über der Flüssigkeitsoberfläche aufgedrückt und nach 4 Stunden Entspannung auf 135 °C erhitzt. Das entstehende Ammoniak wird abgeleitet und in Salzsäure neutralisiert.

Nach Abkühlen auf 90 °C werden 135,2 g (2,5 mol) 85%ige Ameisensäure und 25 g (0,25 mol) konzentrierte Schwefelsäure zugefügt und die Mischung 6 Stunden bei 95 °C gerührt. Nach Abkühlung auf Raumtemperatur wird das Endprodukt abfiltriert, mit reichlich Wasser säurefrei gewaschen und im Vakuum bei 100 °C getrocknet. Man erhält 130,7 g (81,2% d.Th.) 1-Phenyl-1-hydroxy-1,2,4-triazol mit dem Fp. 280–283 °C.

Beispiel 37 (Eintopfverfahren)
1-Phenyl-3-hydroxy-1,2,4-triazol

111,5 g (1 mol) Phenylhydrazin technisch 96%ig, 84 g (1,4 mol) Harnstoff, 71,5 g (1,33 mol) Ammoniumchlorid und 250 ml Wasser werden bis zum Ende der Ammoniakentwicklung (5 Stunden) bei 105–106 °C erhitzt. Nach Abkühlung auf 95 °C werden 135,2 g (2,5 mol) 85%ige Ameisensäure und 33 g (0,3 mol) konzentrierte Salzsäure zugegeben. Die Reaktionsmischung wird 8 Stunden bei dieser Temperatur gerührt, anschliessend wird abgekühlt und der Niederschlag von 1-Phenyl-1,2,4-triazol abgesaugt, mit viel Wasser neutral gewaschen und getrocknet. Man erhält 115,9 g (72% d.Th.) Produkt mit dem Schmelzpunkt 283–284 °C.

**Patentansprüche**

1. Verfahren zur Herstellung von in 1-Stellung substituierten 3-Hydroxy-1,2,4-triazolen durch Umsetzung von gegebenenfalls substituierten Phenylhydrazin(salzen) mit Harnstoff oder Alkali- bzw. Ammoniumcyanaten und Cyclisierung der erhaltenen Phenylsemicarbazide mittels Ameisensäure, dadurch gekennzeichnet, dass man die Cyclisierung in Gegenwart einer anorganischen Säure oder eines Halogenids derselben vornimmt.

2. Verfahren nach Anspruch 1, dadurch gekennzeichnet, dass man die Herstellung und anschliessende Cyclisierung der Phenylsemicarbazide in einer Eintopfreaktion durchführt.

3. Verfahren gemäss Anspruch 1, dadurch gekennzeichnet, dass man Semicarbazide der Formel

$$R\text{–}NH\text{–}NH\text{–}CONH_2,$$

worin

R = ggf. durch ein oder zwei Substituenten aus der Gruppe ($C_1$–$C_4$)-Alkyl, ($C_1$–$C_4$)-Alkoxy, ($C_1$–$C_4$)-Alkylthio, Halogen, Trifluormethyl, Cyano, Nitro und ($C_1$–$C_4$)-Alkoxycarbonyl substituiertes Phenyl bedeutet, cyclisiert.

4. Verfahren nach Ansprüchen 1 bis 3, dadurch gekennzeichnet, dass man als anorganische Säure Salzsäure, Chlorwasserstoffgas, Schwefelsäure oder Phosphorsäure verwendet.

5. Verfahren nach Ansprüchen 1 bis 4, dadurch gekennzeichnet, dass man die anorganische Säure oder deren Halogenid in Konzentrationen von 0,05 bis 0,5, vorzugsweise 0,1–0,3, mol pro mol Semicarbazid anwendet.

6. Verfahren gemäss Ansprüchen 1 bis 5, dadurch gekennzeichnet, dass man die Cyclisierung in einem inerten Lösungsmittel durchführt.

7. Verfahren gemäss Ansprüchen 1 bis 5, dadurch gekennzeichnet, dass man die Cyclisierung in Wasser durchführt.

8. Verfahren gemäss Ansprüchen 1 bis 7, dadurch gekennzeichnet, dass man die Cyclisierung bei Temperaturen von 80° bis 150 °C durchführt.

**Revendications**

1. Procédé de préparation d'hydroxy-3 triazoles-1,2,4- substitués en position 1, par réaction d'une phénylhydrazine éventuellement substituée, ou d'un sel d'une telle phénylhydrazine, avec l'urée ou avec un cyanate de métal alcalin ou d'ammonium, et cyclisation, au moyen de l'acide formique, des phényl-semicarbazides obtenus, procédé caractérisé en ce qu'on effectue la cyclisation en présence d'un acide minéral ou d'un halogénure d'un tel acide.

2. Procédé selon la revendication 1 caractérisé en ce qu'on effectue la préparation des phénylsemicarbazides, puis leur cyclisation, par une réaction en un seul récipient.

3. Procédé selon la revendication 1 caractérisé

en ce qu'on cyclise des semicarbazides répondant à la formule suivante:

$$R{-}NH{-}NH{-}CONH_2$$

dans laquelle R représente un radical phényle éventuellement porteur d'un ou deux substituants pris dans l'ensemble constitué par les radicaux alkyles en $C_1$–$C_4$, les radicaux alcoxy en $C_1$–$C_4$, les radicaux alkylthio en $C_1$–$C_4$, les halogènes, les radicaux trifluorométhyle, cyano et nitro et les radicaux alcoxycarbonyles dont la partie alcoxy contient de 1 à 4 atomes de carbone.

4. Procédé selon l'une quelconque des revendications 1 à 3, caractérisé en ce qu'on utilise, comme acide minéral, l'acide chlorhydrique, le chlorure d'hydrogène gazeux, l'acide sulfurique ou l'acide phosphorique.

5. Procédé selon l'une quelconque des revendications 1 à 4, caractérisé en ce que l'acide minéral, ou son halogénure, est mis en jeu en des concentrations de 0,05 à 0,5 mole par mole du semicarbazide, de préférence en des concentrations de 0,1 à 0,3 mole.

6. Procédé selon l'une quelconque des revendications 1 à 5, caractérisé en ce qu'on effectue la cyclisation dans un solvant inerte.

7. Procédé selon l'une quelconque des revendications 1 à 5, caractérisé se ce qu'on effectue la cyclisation dans l'eau.

8. Procédé selon l'une quelconque des revendications 1 à 7, caractérisé en ce qu'on effectue la cyclisation à des températures de 80 à 150 °C.

## Claims

1. A process for the manufacture of 3-hydroxy-1,2,4-triazoles which are substituted in the 1-position by reacting optionally substituted phenylhydrazines or their salts with urea or alkali or ammonium cyanates and cyclization of the phenylsemicarbazide obtained by means of formic acid, which comprises carrying out the cyclization in the presence of an inorganic acid or of a halide thereof.

2. The process of claim 1, which comprises carrying out the manufacture and subsequent cyclization of the phenylsemicarbazides in a single-pot reaction.

3. The process of claim 1, which comprises cyclizing semicarbazides of the formula

$$R{-}NH{-}NH{-}CONH_2$$

wherein R is phenyl optionally substituted by one or two substituents selected from the group consisting of $(C_1$–$C_4)$alkyl, $(C_1$–$C_4)$alkoxy, $(C_1$–$C_4)$ alkylthio, halogen, trifluoromethyl, cyano, nitro and $(C_1$–$C_4)$alkoxycarbonyl.

4. The process of any of claims 1 to 3, which comprises using as an inorganic acid hydrochloride acid, gaseous hydrogen chloride, sulfuric acid or phosphoric acid.

5. The process of any of claims 1 to 4, which comprises using the inorganic acid or the halide thereof in a concentration of 0.05 to 0.5, preferably 0.1 to 0.3 mol, per mol of semicarbazide.

6. The process of any of claims 1 to 5, which comprises carrying out the cyclization in an inert solvent.

7. The process of any of claims 1 to 5, which comprises carrying out the cyclization in water.

8. The process of any of claims 1 to 7, which comprises carrying out the cyclization at a temperature of 80° to 150 °C.